# EUROPEAN PATENT APPLICATION

(11) **EP 4 253 541 A1**
(43) Date of publication of application: **04.10.2023**
(21) Application number: 21898518.2
(22) Date of filing: 22.11.2021
(51) Int. Cl.: C12N 15/113, C12N 9/22, C12N 15/82, A01H 1/06, A01H 4/00

(54) **METHOD FOR PRODUCING BROWNING-SUPPRESSED POTATO PLANT USING CRISPR/CAS9 SYSTEM**

(30) Priority: 25.11.2020 KR 20200160474
(71) Applicant: Toolgen Incorporated, Seoul 07789 (KR)
(72) Inventor: KWON, Soon Il, Seoul 07789 (KR); HARN, Chee Hark, Seoul 07789 (KR); LEE, Ye Rim, Seoul 07789 (KR); KIM, Ba Reum, Seoul 07789 (KR)
(74) Representative: Cabinet Nony
(86) International application number: PCT/KR2021/017138
(87) International publication number: WO 2022/114692

(57) **Abstract**

The present invention relates to a method for producing a potato plant with suppressed browning by using CRISPR/Cas9 system. More specifically, it relates to a method of producing a genome-edited potato plant with suppressed browning by transfecting a potato protoplast with an RNP (ribonucleoprotein) complex containing sgRNA which targets *StPPO2* (*Solanum tuberosum* Polyphenol Oxidase 2) gene derived from potato.

Since the method of the present invention involves no insertion of a foreign gene and includes only tiny mutation that is hardly distinguishable from natural mutation, it is expected that, unlike GMO (Genetically Modified Organism) crops which require a huge amount of cost and time for evaluating the safety and negative environmental impacts, a great deal of cost and time can be saved in the present invention.

## Description

### Technical Field

The present invention relates to a method for producing a potato plant with suppressed browning using CRISPR/Cas9 system.

### Background Art

In recent years, the trend in consumer food product purchasing indicates a shift of preference to natural products and healthy foods, and simultaneously, due to ever increasing one-person and nuclear family households, there is increasing demand for minimally processed crops which guarantee the freshness and convenience. As the minimally processed crops are provided after being subjected to processes like washing, peeling, cutting, or the like during production stage, they are advantageous in that the consumers can, after making a purchase, use them directly without carrying out a pretreatment process or the like.

Potato is one of the minimally processed crops. However, in accordance with the air exposure of surface and breakdown of cell wall during the processing, enzymatic browning easily occurs in potato.

Main cause of the enzymatic browning in a plant including potato is PPO (polyphenol oxidase) protein, which is an enzyme containing Cu²⁺. The polyphenol compounds present in cell are converted to L-DOPA (L-3,4-dihydroxyphenylalanine) according to oxidization by PPO. L-DOPA is subsequently converted to DOPA quinone and forms, after an oxidative polymerization, a melanin pigment with dark brown color to exhibit the browning phenomenon.

The browning reaction is known to cause undesirable color and smell of minimally processed crops and also to lower the nutritional value of the crops. In addition, as the product undergone browning during storage and transport of minimally processed crops has a low commercial value to yield economic loss, it is quite necessary to suppress the browning.

The genome editing technique in recent years suggests a possibility of inducing genetic mutation at target genomic DNA site by using site-specific nucleases of various types. In particular, in relation with plants and other various organisms, CRISPR/Cas9 system is known to be a potent tool for genome editing. The advantage of CRISPR/Cas9 system is that it allows accurate and efficient introduction of mutation to a target site. It is also shown that the genome edited crops are not different from the crops which have been developed by a traditional breeding technique.

Meanwhile, in Japanese Patent Application Publication No. 2019-507595, "Plant showing a reduced wound-induced surface discoloration" is disclosed, and, in Korean Patent Application Publication No. 2016-0087651, "Browning prevented colored potato and method for preventing browning of colored potato" is disclosed. However, "Method for producing a potato plant with suppressed browning using CRISPR/Cas9 system" has not been described before.

### Detailed Description of the Invention

### Technical Problems to be Solved

The present invention is devised under the circumstances that are described in the above. To suppress the browning by editing *StPPO2* (*Solanum tuberosum* Polyphenol Oxidase 2) gene using CRISPR system instead of common cross breeding or gene modification method, inventors of the present invention introduced RNP complex (*StPPO2* sgRNA+Cas9) by PEG-mediated transfection after isolating protoplast from young germ-free potato plant obtained by *in vitro* culture, and eventually selected a plant with edited *StPPO2* gene based on cell reproduction and generation of the protoplasts which have been introduced with the complex. Further, by choosing a potato line with suppressed activity of PPO2 protein and suppressed browning from the gene-edited plants obtained after the above selection, the inventors accordingly completed the present invention.

### Technical Meals for Solving Problems

To achieve the object described in the above, the present invention provides a composition for genome editing to suppress browning in a potato plant comprising, as an active ingredient, an ribonucleoprotein complex of a guide RNA specific to the target nucleotide sequence in *StPPO2* gene derived from potato and an endonuclease protein; or a recombination vector comprising a DNA encoding a guide RNA specific to the target nucleotide sequence in *StPPO2* gene derived from potato and a nucleic acid sequence encoding an endonuclease protein.

The present invention further provides a guide nucleic acid which is an RNA sequence specific to a target region in the nucleotide sequence constituting *StPPO2* gene, in which the target region is the nucleotide sequence of SEQ ID NO: 5.

The present invention further provides a method of producing a genome-edited potato plant with suppressed browning including steps of (a) introducing a guide RNA specific to the target nucleotide sequence in *StPPO2* gene derived from potato and an endonuclease protein to a potato plant cell to have genome editing; and (b) regenerating a potato plant from the potato plant cell that is genome-edited.

The present invention still further provides a genome-edited potato plant with suppressed browning which is produced by the aforementioned method, and genome-edited seed potato thereof.

### Advantageous Effect of the Invention

The genome-edited potato plant with suppressed browning produced by the method of the present invention has delayed browning even when it is previously cut or mashed, and thus it can be conveniently used for processing or cooking and additional processes like heating or adding an artificial additive for suppressing the browning can be minimized. In addition, as the method of the present invention involves no insertion of a foreign gene and includes only tiny mutation that is hardly distinguishable from natural mutation, it is expected that, unlike GMO (Genetically Modified Organism) crops which require a huge amount of cost and time for evaluation of the safety and negative environmental impacts, a great deal of cost and time can be saved in the present invention.

### Best Description of Drawings

FIG. 1 to FIG. 4 show the result of the target deep-sequencing in *in vitro* assay which has been carried out for determining candidate sgRNA.
FIG. 5 shows the separation of protoplast from the leaf of a young potato plant, in which (A) represents VCP treatment of the leaf of a young potato plant, (B) represents the intact protoplast (indicated with red arrow) which has been determined by separation of the VCP-treated protoplast based on sucrose concentration gradient, and (C) represents a photographic image of the protoplast under microscope which has been finally separated.
FIG. 6 shows the photographic image of a plant regenerated from a potato protoplast which has been introduced with CRISPR/Cas9 RNP complex.
FIG. 7 to FIG. 10 show the result of the deep-sequencing of several plants from the plants regenerated from a protoplast which has been introduced with CRISPR/Cas9 RNP complex.
FIG. 11 shows the result of *StPPO2* editing efficiency in the potato protoplast obtained according to the method of the present invention (left column), and the InDel pattern of *StPPO2* edited product derived from the potato protoplast (right column).
FIG. 12 shows the result of measuring the activity of PPO2 protein in which the measurement was carried out by using potato tuber.
FIG. 13 shows the result of observing the browning of potato tuber, in which (A) represents the result of comparing, after slicing potato tuber, the control (WT) with the gene edited product (GE#14, #25), and (B) represents the result of comparing, after removing only the skin of potato tuber, the control (WT) with the gene edited product (GE#14, #25).

### Best Mode (s) for Carrying out the Invention

To achieve the object of the present invention, the present invention provides a composition for genome editing to suppress browning in a potato plant comprising, as an active ingredient, an ribonucleoprotein complex of a guide RNA specific to the target nucleotide sequence in *StPPO2* gene derived from potato and an endonuclease protein; or a recombination vector comprising a DNA encoding a guide RNA specific to the target nucleotide sequence in *StPPO2* gene derived from potato and a nucleic acid sequence encoding an endonuclease protein.

In an embodiment of the composition of the present invention, the target gene for genome editing to suppress the browning in potato plant is *StPPO2* gene which consists of the nucleotide sequence of SEQ ID NO: 1.

As described herein, the term "genome/gene editing" means a technique for introducing a target-oriented mutation to a genome nucleotide sequence of a plant or an animal cell including human cell. Specifically, it indicates a technique for knock-out or knock-in of a specific gene by deletion, insertion, or substitution of one more nucleic acid molecules by DNA cutting, or a technique for introducing a mutation even to a non-coding DNA sequence which does not produce any protein. According to the purpose of the present invention, the genome editing may be an introduction of a mutation to a plant by using an endonuclease, for example, Cas9 (CRISPR associated protein 9) protein, and a guide RNA. Furthermore, the term "gene editing" may be interchangeably used with "gene engineering".

Furthermore, the term "target gene" means part of DNA present in the genome of a plant to be edited according to the present invention. Type of the target gene is not limited, and it may include a coding region and also a non-coding region. Depending on the purpose, a person who is skilled in the pertinent art may select the target gene according to the mutation of a plant desired to be produced by genome editing.

Furthermore, the term "guide RNA" means a ribonucleic acid which includes RNA specific to a target DNA in nucleotide sequence encoding the target gene and, according to complementary binding between the whole or partial sequence of the guide RNA and the nucleotide sequence of target DNA, the guide RNA plays the role of guiding an endonuclease to the target DNA nucleotide sequence. The guide RNA indicates RNA in single guide RNA (sgRNA) form comprising the first site which includes a dual RNA having two RNAs, i.e., crRNA (CRISPR RNA) and tracrRNA (trans-activating crRNA), as a constitutional element; or a sequence which is fully or partially complementary to the nucleotide sequence in the target gene, and the second site which includes a sequence interacting with an endonuclease (in particular, RNA-guide nuclease), but those in the form in which the endonuclease is active for the target nucleotide sequence are also within the scope of the invention without any limitation. Considering the type of an endonuclease to be used together, microorganisms from which the endonuclease is derived, or the like, the guide RNA may be used after being prepared in consideration of a method well known in the pertinent art.

Furthermore, the guide RNA may be those transcribed from a plasmid template, those obtained by *in vitro* transcription (e.g., oligonucleotide double strand), or those obtained by synthesis, or the like, but it is not limited thereto.

In an embodiment of the composition for genome editing of the present invention, the guide RNA is devised to have specificity for the target nucleotide sequence in *StPPO2* gene consisting of the nucleotide sequence of SEQ ID NO: 5, in which the guide RNA devised to have specificity for the nucleotide sequence of *StPPO2* gene is characterized in that it has higher efficiency of inducing InDel mutation compared to other guide RNAs for editing *StPPO2* gene that are devised to have specificity for the target nucleotide sequence of SEQ ID NO: 2 to SEQ ID NO: 4. In particular, because potato is a tetraploid crop, when CRISPR/Cas system is applied to potato by using a RNA guide having high InDel efficiency, it is likely to have higher efficiency of obtaining individual potato plant in which all four alleles are edited.

Furthermore, in an embodiment of the composition for genome editing of the present invention, the endonuclease protein may be one or more selected from a group consisting of Cas9, Cpf1 (CRISPR from Prevotella and Francisella 1), TALEN (Transcription activator-like effector nuclease), ZFN (Zinc Finger Nuclease), and a functional analog thereof. It may be preferably Cas9 protein, but it is not limited thereto.

Furthermore, the Cas9 protein may be one or more selected from a group consisting of Cas9 protein derived from *Streptococcus pyogenes,* Cas9 protein derived from *Campylobacter jejuni,* Cas9 protein derived from *S*. *thermophilus,* Cas9 protein derived from *S*. *aureus,* Cas9 protein derived from *Neisseria meningitides,* Cas9 protein derived from *Pasteurella multocida,* Cas9 protein derived from *Francisella novicida,* and the like, but it is not limited thereto. Information of Cas9 protein or gene of Cas9 protein can be obtained from a known database like GenBank of NCBI (National Center for Biotechnology Information).

Cas9 protein is an RNA-guided DNA endonuclease enzyme which induces breakage of a double stranded DNA. In order for Cas9 protein to cause DNA breakage after precise binding to a target nucleotide sequence, a short nucleotide sequence consisting of three nucleotides, which is known as PAM (Protospacer Adjacent Motif), should be present next to the target nucleotide sequence, and Cas9 protein causes the breakage by assuming the position between the 3^{rd} and the 4^{th} base pairs from the PAM sequence (NGG).

In an embodiment of the composition for genome editing of the present invention, the guide RNA and endonuclease protein may function as RNA gene scissors (RNA-Guided Engineered Nuclease, RGEN) by forming a ribonucleic acid-protein (i.e., ribonucleoprotein) complex.

The present invention further provides a guide nucleic acid specific to a target region in the nucleotide sequence constituting *StPPO2* gene, in which the target region is the nucleotide sequence of SEQ ID NO: 5.

The "guide nucleic acid" according to the present invention means the aforementioned "guide RNA", and it includes an endonuclease binding site in addition to the RNA sequence specific to the target region. The endonuclease may be preferably an RNA-guide nuclease, but it is not limited thereto.

The guide nucleic acid of the present invention may be preferably in the form of a single guide RNA (sgRNA), but it is not limited thereto.

According to the guide nucleic acid of one embodiment of the present invention, the RNA sequence specific to a target region may be synthesized by replacing adenine (A), thymine (T), or cytosine (C) of the target sequence present at the 20^{th} position from the PAM nucleotide with guanine (G), but it is not limited thereto.

The guide nucleic acid according to the present invention is characterized in that, when an insertion-deletion (InDel) mutation is induced in a target sequence of *StPPO2* gene consisting of the nucleotide sequence of SEQ ID NO: 5, deletion mutation with size of several base pairs (i.e., 1 to 10 bp) is caused. Thus, in terms of the genome mutation, it has relatively higher safety than a guide RNA which may cause an insertion or a deletion mutation with size of several hundred base pairs.

The present invention further provides a method of producing a genome-edited potato plant with suppressed browning including (a) introducing a guide RNA specific to the target nucleotide sequence in *StPPO2* gene derived from potato and an endonuclease protein to a potato plant cell to have genome editing; and (b) regenerating a potato plant from the potato plant cell that is genome-edited.

According to the production method of one embodiment of the present invention, the guide RNA specific to the target nucleotide sequence in *StPPO2* gene and endonuclease protein are as defined in the above.

The CRISPR/Cas9 system employed in the present invention is a method of gene editing based on NHEJ (non-homologous end joining) mechanism in which insertion-deletion (InDel) mutation resulting from incomplete repair, which is induced during a process of DNA repair, by introducing breakage of a double strand at specific site of a specific gene to be edited.

In an embodiment of the production method of the present invention, the aforementioned step (a) for introducing a guide RNA and an endonuclease protein to a potato plant cell may use an ribonucleoprotein complex of a guide RNA specific to the target nucleotide sequence in *StPPO2* gene derived from potato and an endonuclease protein; or a recombination vector comprising a DNA encoding a guide RNA specific to the target nucleotide sequence in *StPPO2* gene derived from potato and a nucleic acid sequence encoding an endonuclease protein, but it is not limited thereto.

In an embodiment of the production method of the present invention, the transfection method for introducing the complex of a guide RNA and an endonuclease protein to a plant cell may be appropriately selected from a calcium/polyethylene glycol method for protoplasts (Krens, F.A. et al., 1982, Nature 296, 72-74; Negrutiu I. et al., June 1987, Plant Mol. Biol. 8, 363-373), an electroporation method for protoplasts (Shillito R.D. et al., 1985 Bio/Technol. 3, 1099-1102), a microscopic injection method for plant components (Crossway A. et al., 1986, Mol. Gen. Genet. 202, 179-185), a (DNA or RNA-coated) particle bombardment method for various plant components (Klein T.M. et al., 1987, Nature 327, 70), or a (non-complete) viral infection method in *Agrobacterium tumefaciens* mediated gene transfer by plant invasion, or the like.

Furthermore, the introduction of a recombination vector comprising a DNA encoding a guide RNA specific to the target nucleotide sequence and a nucleic acid sequence encoding an endonuclease protein means transformation. Transformation of a plant species is now generally carried out for all plant species including not only a dicot plant but also a monocot plant. In principle, any method for transformation may be used for introducing the recombination vector of the present invention to a suitable progenitor cell.

Furthermore, in an embodiment of the production method of the present invention, the "plant cell" to which a guide RNA specific to the target nucleotide sequence and an endonuclease are introduced may be any type of a plant cell. The plant cell may be a cultured cell, a cultured tissue, a cultured organ, or a whole plant. The "plant tissue" is either a generated tissue or a non-generated tissue of a plant, for example, root, stem, leaf, pollen, small spore, egg cell, seed, and cells in various form that are used for culture, i.e., single cell, protoplast, shoot, and callus tissue, although it is not limited thereto. The plant tissue may be an in planta tissue or in the form of cultured organ, cultured tissue, or cultured cell. The plant cell preferred in the present invention is a protoplast.

In an embodiment of the production method of the present invention, the potato plant cell of step (b) which is obtained after genome editing may be a cell in which deletion mutation of 1 to 10 bp in size has been induced in the target nucleotide sequence in *StPPO2* gene, but it is not limited thereto.

In an embodiment of the production method of the present invention, any method well known in the pertinent art can be used as a method of regenerating a genome-edited plant from a plant cell with edited genome. A plant cell with edited genome needs to be regenerated to a whole plant. Techniques for regeneration into a mature plant from culture of callus or protoplast are well known in the pertinent art for various types of plants (Handbook of Plant Cell Culture, volume 1 to 5, 1983-1989 Momillan, N.Y.).

The present invention still further provides a genome-edited potato plant with suppressed browning which is produced by the aforementioned method, and genome-edited seed potato thereof.

In the genome-edited potato plant with suppressed browning of the present invention, *StPPO2* gene involved in browning is edited by using CRISPR/Cas9 system, and it is a genome-edited potato plant which has, due to the knock-out of *StSPPO2* gene derived from potato, a trait with suppressed browning compared to a potato plant without any genome editing.

In particular, the genome-edited potato plant of the present invention may have a deletion mutation of 1 to 10 bp in size that is induced in the target nucleotide sequence in *StPPO2* gene, and it is characterized by having, in terms of the genome mutation, relatively higher safety than a genome-edited plant in which an insertion or a deletion mutation of several hundred base pairs in size has been made.

As described herein, the term *"StPPO2"* may be interchangeably used with the term *"StuPPO2".*

Hereinbelow, the present invention is explained in greater detail in view of the Examples. However, the following Examples are given only for exemplification of the present invention and the scope of the present invention is not limited by them.

### EXAMPLES

### Example 1. Selection of StPPO2 gene sgRNA

Four different sgRNAs were selected from the website of CRISPR RGEN Tools (www.rgenome.net/Cas-designer) by using the sequence of potato *StPPO2* gene (Table 1). The sgRNAs described in the following Table 1 have been selected by considering various conditions such as GC contents, out-of-frame score, mismatch number in genome sequence, or the like. In the present invention, the nucleotide sequences of SEQ ID NO: 2 to 5 represent the sequence of RGEN target sequence of the following Table 1 excluding the PAM base (underlined).

**Table 1**

| sgRNA Target sequence for editing potato *StPPO2* gene | | | | | | | |
|---|---|---|---|---|---|---|---|
| sgRNA | RGEN target* (5'→3') (SEQ ID NO) | Direction | GC content (%, w/o PAM) | Out-of-frame score | Mismatc hes | | |
| | | | | | 0 | 1 | 2 |
| StPPO2-1 | TCCATGGATGAAAAGTTG AGAGG (SEQ ID NO: 2) | - | 40 | 91.2 | 0 | 2 | 1 |
| StPPO2-2 | TGCATGAAACTTTGAACA | - | 30 | 75.0 | 2 | 0 | 0 |
| | TTTGG (SEQ ID NO: 3) | | | | | | |
| StPPO2-3 | ATACTACAAGACGAGAGA TCAGG (SEQ ID NO: 4) | - | 40 | 79.1 | 0 | 0 | 2 |
| StPPO2-4 | TTAGGCGCGCAACAACTG TACGG (SEQ ID NO: 5) | - | 50 | 72.5 | 1 | 0 | 0 |
| * underlined; PAM base | | | | | | | |

After synthesizing each of the four selected sgRNAs against target sequence, *in vitro* cleavage assay and *in vivo* assay were carried for their determination. *In vitro* cleavage assay is an assay for examining the activity of sgRNA against target DNA in which a test tube containing PCR fragments of target DNA is added with a sample not containing any Cas9 and sgRNA (sample 1), a sample containing Cas9 and sgRNA at ratio of 1 : 1 (sample 2), or a sample containing Cas9 and sgRNA at ratio of 1 : 3 (sample 3) followed by reaction and the reaction product is analyzed by electrophoresis. When sgRNA used for the analysis acts on the target RNA, the target DNA is broken to exhibit at least 2 bands. On the other hand, when sgRNA the does not act on the target RNA, no breakage product is identified.

Furthermore, *in vivo* assay is an assay for determining InDel efficiency in which each of the selected sgRNAs is introduced, together with Cas9, by PEG-mediated transfection to protoplast cells (5 × 10⁵), and after 3 days or so (i.e., time for RNP [Cas9+sgRNA] complex to have a reaction with the target gene in nucleus), genomic DNA is isolated from the protoplast and target deep-sequencing is carried out by PCR.

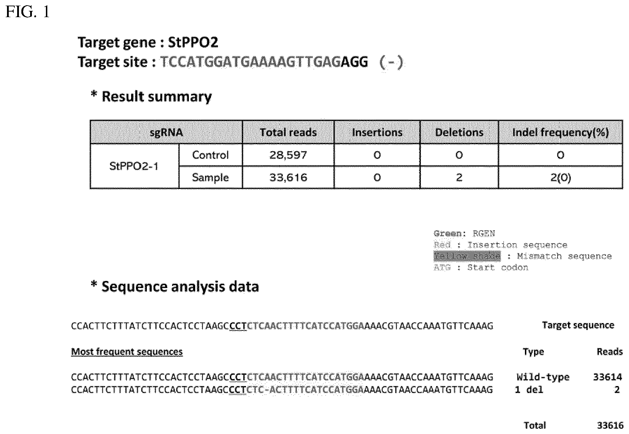

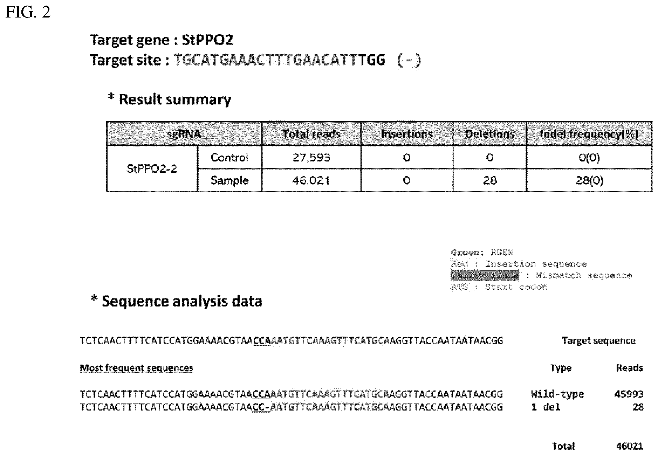

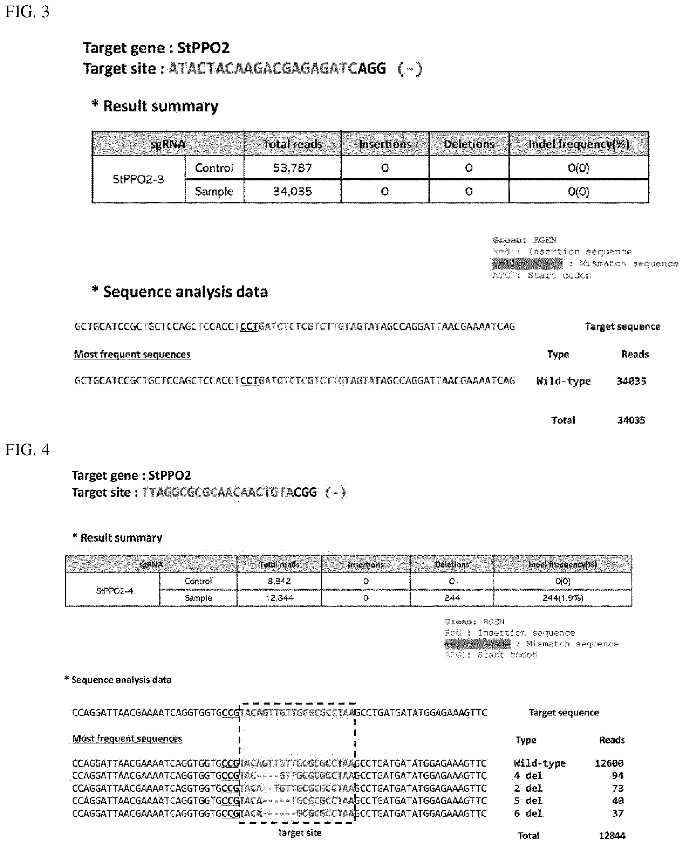

Four sgRNAs selected by *in vivo* assay are found to have InDel efficiency of 0% for StPPO2-1 to StPPO2-3, and 1.9% for StPPO2-4 (FIGs. 1 to 4). Based on these assay results, the inventors of the present invention finally selected StPPO2-4 (SEQ ID NO: 5) as a target sequence of sgRNA for *StPPO2* gene editing.

### Example 2. Separation of potato protoplast and method of culturing potato protoplast

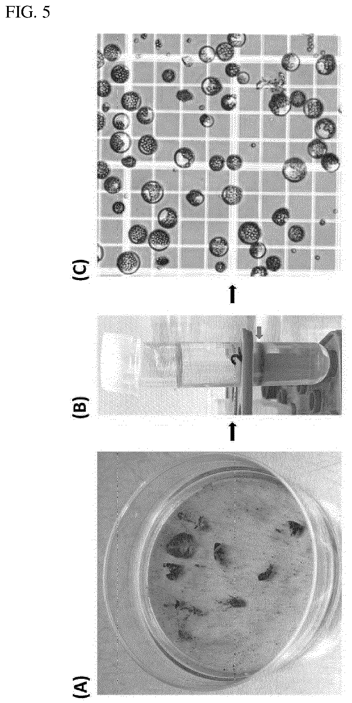

To a petri dish, VCP (Viscozyme, Celluclast, PectinEX) enzyme solution (10 ml) was added. About 5 to 7 leaves of young potato plant of Desiree variety (*Solanum tuberosum* L. cv. Desiree) which has been obtained by *in vitro* culture were cut and added to the petri dish such that the backside of leaf faces is on the top. Then, the reaction was allowed to occur for 5 hours or so at 25°C and stirring speed of 45 rpm under dark conditions. After making an observation of the degree of enzyme degradation, when it is found that the leaf tissues turn to be transparent, separation of protoplast was carried out. Once the VCP enzyme reaction is completed, the mixture was filtered using a disposable mesh (Falcon, 40 *µ*m), and the resultant was transferred to a 14 ml round tube and centrifuged for 5 minutes at 800 rpm. After the centrifuge, the supernatant was discarded. W5 solution (Menczel L. et al. (1981) Theor. Appl. Genet. 59; 191-195) was added (1 ml) and the protoplast was carefully dissolved. After adding slowly 9 ml of the W5 solution thereto, the resultant was centrifuged for 5 minutes at 800 rpm to have first washing. By repeating the same procedure as above, second washing was carried out. Thereafter, the supernatant was discarded and 5 ml of the W5 solution was slowly added, and the resultant was carefully transferred to a 15 ml round tube which has been added with 20% sucrose (5 ml) and centrifuged for 5 minutes at 800 rpm. After the centrifuge, a protoplast layer (i.e., green band) was seen near the middle part of the tube while the broken protoplast or part of the tissues was found to be settled at the bottom of the tube. Using a blunt-end pipette tip, the protoplast layer was carefully extracted, which was then transferred to a fresh 15 ml round tube added with 10 ml of the W5 solution, and centrifuged for 5 minutes at 800 rpm. The supernatant was discarded after the centrifuge and the protoplast was dissolved well by adding 1 ml of the W5 solution. Then, the state of the protoplast was observed under a microscope (FIG. 5).

If the separated protoplast is in good state, it was added to a 6-well plate with PIM (Protoplast Inducing Media, 3 ml), and the protoplast and cell division pattern was observed every day. Cell division is generally observed after 5 days or so. After 2 weeks or so, cell accumulation starts to appear so that microcalli (observed with a naked eye, less than 1 mm) are observed after 3 weeks or so. After 4 weeks or so, it is possible to observe with a naked eye white spots in culture using 1.2% low melting agar.

**Table 2**

| **Composition of solution used for protoplast separation** | | | | |
|---|---|---|---|---|
| [Method for preparing CPW solution] | | | | |
| | Composition | **Stock name** | | 1X CPW concentration **(Final Conc.)** |
| **CPW salts** | **CaCl₂-2H₂O** | **Stock β** | | 1480 mg/L |
| | **KH₂PO₄** | **Stock A** | | 27.2 mg/L |
| | **KNO₃** | | | 101 mg/L |
| | **MgSO₄-7H₂O** | | | 246 mg/L |
| | **Kl** | | | 0.16 mg/L |
| | **C_{U}SO₄-5H₂O** | | | 0.025 mg/L |
| | | | | **pH 5.7** |
| - Stock A solution was prepared as 100X solution and used after dilution to appropriate concentration | | | | |
| - Stock B solution was prepared as 10X solution and used after dilution to appropriate concentration | | | | |
| | | | | |

| [Method for preparing VCP solution] | | | | |
|---|---|---|---|---|
| Composition | | | Content (for preparing 500 ml) | |
| **CPW stock A (X 100)** | | | 5 ml | |
| **CPW stock B (X 10)** | | | 50 ml | |
| **Mannitol** | | | 45 g | |
| **MES buffer (A)** | | | 533 mg | |
| **Viscozyme (B)** | | | 5 ml | |
| **Celludast (C)** | | | 2.5 ml | |
| **PectinEX** | | | 2.5 ml | |
| | | | **pH 5.7** | |
| - Prepared solution was subjected to sterile filtration at 0.22 um pore size without any autoclave, and stored in refrigerator until use | | | | |

### Example 3. Gene editing of potato protoplast using CRISPR/Cas9 RNP complex

The protoplast separated by the method described in Example 2 was counted using a hemocytometer under a microscope. About 100,000 protoplasts were used for the test of CRISPR/Cas9 gene editing.

After completing the protoplast separation, CRISPR/Cas9 RNP complex was produced as follows. First, 15 *µ*g of StPPO2-4 sgRNA (sgRNA of 5 *µ*g/*µ*ℓ concentration was used in an amount of 3 *µ*ℓ) and 30 *µ*g of Cas9 (Cas9 protein of 5 *µ*g/*µ*ℓ concentration was used in an amount of 6 *µ*ℓ) were added to a 2 ml tube, added with 1 *µ*ℓ of NEB buffer (#3), and then reacted for 10 minutes at room temperature. Thereafter, CRISPR/Cas9 RNP complex was introduced to the prepared protoplast using PEG-mediated transfection. Namely, the protoplast (1 × 10⁵, 200 *µ*ℓ) was admixed with CRISPR/Cas9 RNP complex (10 *µ*ℓ), and after adding 40% PEG solution in the same volume as the mixture (i.e., 210 *µ*ℓ), the reaction was allowed to occur for 10 minutes at room temperature. Upon the completion of the reaction after 10 minutes, the W5 solution in the same volume as the mixture (i.e., 420 *µ*ℓ) was added for having first washing followed by centrifuge for 5 minutes at 600 rpm. The supernatant was discarded after the centrifuge. After second washing by adding again the W5 solution (1 ml) to remove residual PEG, centrifuge was carried out at the same condition as above. During the washing, PIM was aliquoted in advance in an amount of 3 ml to a 6-well plate. To the protoplast from which the supernatant has been removed after the centrifuge, 1 ml PIM solution was added and mixed well. The mixture was then aliquoted in an amount of 500 *µ*ℓ to each well of the 6-well plate followed by sealing. Then, culture was carried out at 25°C, dark condition and the state of protoplast was examined.

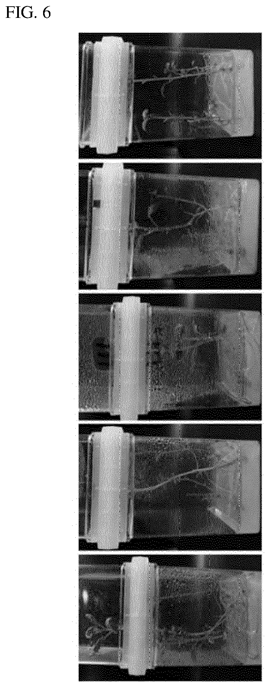

Once the protoplast shows relatively stable division after 2 weeks or so, it was added with low melting agar with concentration of 1.2% followed by solidification. Then, observation was made under culture at the same condition. After 3 weeks or so following the introduction of CRISPR/Cas9 RNP complex, microcalli starts to appear and the microcalli under culture in low melting agar/PIM were transferred to CFM (Callus Forming Media) and continuously cultured therein. Callus under culture in CFM (diameter of about 0.5 cm) was transferred to SIM (Shoot Inducing Media) and culture therein. To induce roots from a plant with shoots, transfer to RIM (Root Inducing Media) was made followed by culture therein. It was found that, after 16 weeks or so, the protoplast introduced with CRISPR/Cas9 RNP complex is fully grown to a single plant (FIG. 6). Meanwhile, with regard to the composition of the media including PIM, CFM, SIM, and RIM which have been used for protoplast culture described above, reference was made to the report by Ehsanpour and Jones (J. Sci. I. R. Iran. (2001), 12 (2): 103-110).

**Table 3**

| Composition of PEG solution for transfection | |
|---|---|
| 40% PEG Solution | For preparing 2.5 ml |
| PEG 4000 | 1g |
| 1M Mannitol | 0.5 ml |
| 1M CaCl₂ | 0.25 ml |
| ddH₂0 | To be 2.5 ml (about 1 ml or so) |
| After addition to a 14 ml round tube and adjustment to 2.5 ml, it was placed in a microwave for 20 seconds, mixed well to have full dissolving, cooled down at room temperature, filtered, and used. | |
| Fresh PEG solution was used bv preparing it at each time of the experiment | |

### Example 4. Selection of StPPO2 edited product derived from potato protoplast

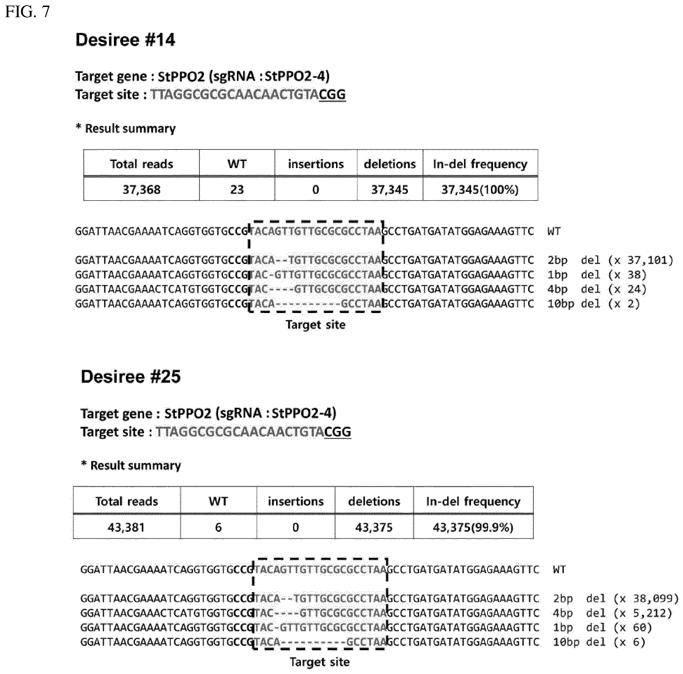

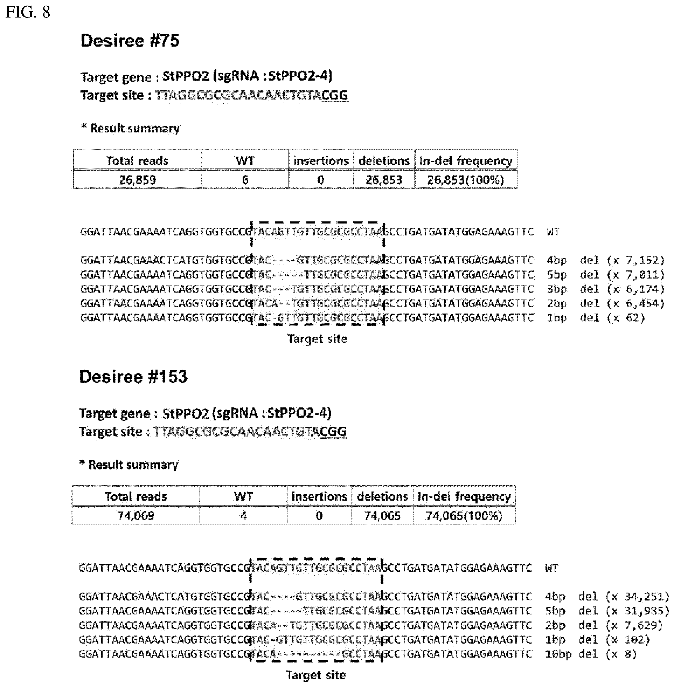

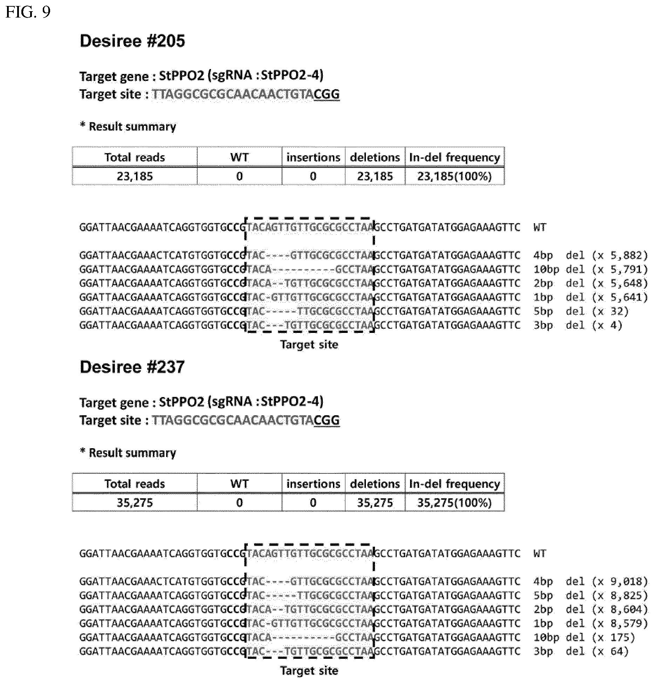

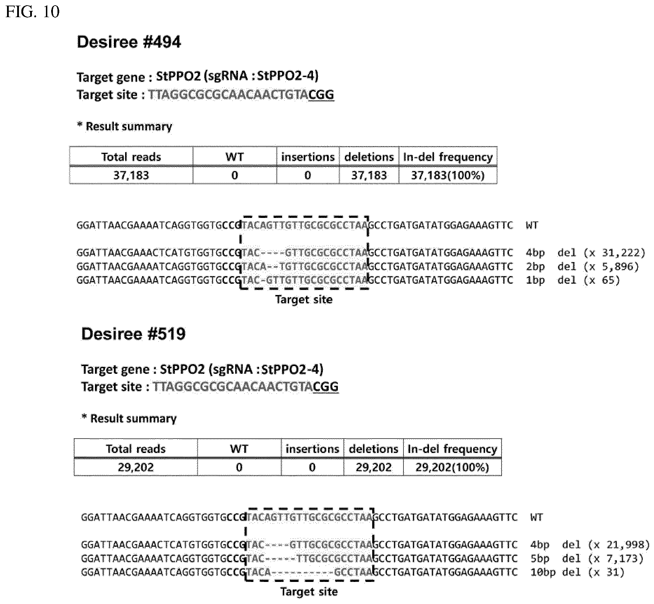

For 590 plants of the total 640 plants obtained from Example 3, editing of *StPPO2* gene was examined by deep-sequencing. As a result, it was finally found that the gene editing is induced in at least one allele in 110 plants while 3 plants show that the 4 alleles are edited in all different InDel patterns. FIGs. 7 to 10 illustrate the result of deep-sequencing of some of those plants. The result of deep-sequencing of the total 110 plants, which have been finally found to have edited *StPPO2* gene, is the same as those described in Table 4.

**Table 4**

| Result of deep-sequencing of plants which have been found to have edited *StPPO2* gene | | | | | | | | | | | | |
|---|---|---|---|---|---|---|---|---|---|---|---|---|
| Target gene | Sample | Total reads | WT | Insertions | Deletions | In-del frequency (%) | Sample | Total reads | WT | Insertions | Deletions | In-del frequency (%) |
| | 13 | 16373 | 84 | 10265 | 6024 | 16298 (99.5%) | 323 | 18987 | 0 | 0 | 18987 | 18987( 100%) |
| | 14 | 37368 | 203 | 0 | 37165 | 37165 (99.9%) | 331 | 15570 | 0 | 0 | 15570 | 15570(100%) |
| | 16 | 41402 | 22 | 0 | 41380 | 41380 (99.9%) | 338 | 38796 | 0 | 0 | 38796 | 38796(100%) |
| | 24 | 47184 | 91 | 0 | 47093 | 47093 (99.9%) | 346 | 28216 | 0 | 0 | 28216 | 28216(100%) |
| | 25 | 43381 | 6 | 0 | 43375 | 43375 (99.9%) | 350 | 36566 | 0 | 0 | 36566 | 36566(100%) |
| | 30 | 21108 | 73 | 0 | 21035 | 21035(99.8%) | 353 | 18016 | 0 | 0 | 18016 | 18016(100%) |
| | 31 | 25462 | 19 | 0 | 25443 | 25443 (99.9%) | 355 | 36073 | 0 | 0 | 36073 | 36073(100%) |
| | 38 | 16599 | 0 | 0 | 16599 | 16599 (100%) | 356 | 10496 | 0 | 0 | 10496 | 10496(100%) |
| | 50 | 16485 | 0 | 0 | 16485 | 16485 (100%) | 365 | 28178 | 0 | 0 | 28178 | 28178(100%) |
| | 51 | 21638 | 0 | 0 | 21638 | 21633 (100%) | 371 | 26021 | 0 | 0 | 26021 | 26021(100%) |
| | 52 | 11835 | 0 | 0 | 11835 | 11835 (100%) | 376 | 25475 | 0 | 0 | 23475 | 23475(100%) |
| | 62 | 32061 | 0 | 0 | 32059 | 32059 (100%) | 380 | 28852 | 0 | 0 | 28852 | 28852(100%) |
| | 69 | 31237 | 15 | 0 | 31222 | 31222(99.9%) | 390 | 16213 | 8 | 0 | 16205 | 16205(100%) |
| | 75 | 26859 | 6 | 0 | 26853 | 26853(100%) | 192 | 13176 | 0 | 0 | 13176 | 13176(100%) |
| | 83 | 45162 | 19 | 0 | 45143 | 45143(99.9%) | 395 | 19293 | 0 | 0 | 19293 | 19293(100%) |
| | 89 | 14081 | 0 | 0 | 14081 | 14081 (100%) | 406 | 21283 | 0 | 0 | 21283 | 21283(100%) |
| | 93 | 32996 | 0 | 0 | 32996 | 32996(100%) | 407 | 24463 | 0 | 0 | 24463 | 24463(100%) |
| | 102 | 26501 | 2 | 0 | 26499 | 26499(100%) | 413 | 31737 | 0 | 0 | 31737 | 31737(100%) |
| | 112 | 37448 | 0 | 0 | 37448 | 37448(100%) | 414 | 12820 | 0 | 0 | 12820 | 12820(100%) |
| | 127 | 26125 | 0 | 0 | 26125 | 26125(100%) | 418 | 12600 | 0 | 0 | 12609 | 12609 ( 100%) |
| | 128 | 42639 | 0 | 0 | 42639 | 42639(100%) | 427 | 38992 | 0 | 0 | 38 892 | 3889 2 (100%) |
| | 130 | 43563 | 0 | 0 | 43563 | 43563(100%) | 433 | 12647 | 0 | 0 | 12647 | 12647(1 00)%) |
| | 132 | 43 21 2 | 0 | 0 | 43212 | 43212(100 %) | 440 | 25470 | 0 | 0 | 25470 | 25470(100 %) |
| | 136 | 6995 5 | 0 | 0 | 69955 | 69955(100 %) | 447 | 26169 | 0 | 0 | 26169 | 26169(100%) |
| | 150 | 50291 | 0 | 0 | 50291 | 50291(100%) | 448 | 31817 | 0 | 0 | 31817 | 31817(100%) |
| | 153 | 74069 | 4 | 0 | 74065 | 74065(100%) | 461 | 19279 | 0 | 0 | 19279 | 19279(100%) |
| | 159 | 44769 | 0 | 0 | 44769 | 44769(100%) | 466 | 13381 | 0 | 0 | 13381 | 13381(100%) |
| StuPPO2-4 | 161 | 44838 | 2 | 0 | 44836 | 44836(100%) | 470 | 18672 | 0 | 0 | 18672 | 18672(100%) |
| | 165 | 54908 | 2 | 0 | 54906 | 54906(100%) | 472 | 10305 | 0 | 0 | 10305 | 10305(100%) |
| | 166 | 24901 | 0 | 0 | 24901 | 14901(100%) | 484 | 22718 | 0 | 0 | 22718 | 22718(100%) |
| | 174 | 29221 | 0 | 0 | 29221 | 29221(100%) | 494 | 37183 | 0 | 0 | 37183 | 37183(100%) |
| | 175 | 23590 | 0 | 0 | 23590 | 23590(100%) | 49d | 33914 | 0 | 0 | 33914 | 33914(100%) |
| | 176 | 55272 | 0 | 0 | 55272 | 55272(100%) | 502 | 29990 | 0 | 0 | 29990 | 29990(100%) |
| | 180 | 32055 | 0 | 0 | 32055 | 32055(100%) | 503 | 28795 | 0 | 0 | 28795 | 28795(100%) |
| | 183 | 3970 4 | 0 | 0 | 39704 | 39704(100%) | 505 | 31215 | 0 | 0 | 31215 | 31215( 100%) |
| | 186 | 34078 | 0 | 0 | 34078 | 34078(100%) | 509 | 25079 | 0 | 0 | 25079 | 2 5079( 100%) |
| | 195 | 41677 | 2 | 0 | 41675 | 41675(100%) | 512 | 38333 | 0 | 0 | 38333 | 38333( 100%) |
| | 197 | 19526 | 0 | 0 | 19526 | 19526(100%) | 514 | 29645 | 0 | 0 | 29645 | 29645(100%) |
| | 205 | 23185 | 0 | 0 | 23185 | 23185(100%) | 519 | 29202 | 0 | 0 | 29202 | 29202(100%) |
| | 209 | 54624 | 0 | 0 | 54624 | 54624(100%) | 529 | 23519 | 0 | 0 | 23519 | 23519(100%) |
| | 212 | 41742 | 0 | 0 | 41742 | 41742(100%) | 530 | 34261 | 0 | 0 | 34261 | 34261(100%) |
| | 229 | 18983 | 0 | 0 | 18983 | 18983(100%) | 534 | 31146 | 0 | 0 | 31146 | 31146(100%) |
| | 231 | 10076 | 0 | 0 | 10076 | 10076(100%) | 542 | 28272 | 0 | 0 | 28272 | 28272(100%) |
| | 237 | 35275 | 0 | 0 | 35275 | 35275(100%) | 543 | 31396 | 0 | 0 | 31396 | 31396(100%) |
| | 239 | 30244 | 0 | 0 | 30244 | 30244(100%) | 544 | 39558 | 0 | 0 | 39558 | 39558( 100%) |
| | 255 | 58667 | 0 | 0 | 58667 | 58667(100%) | 548 | 18958 | 0 | 0 | 18958 | 18958( 100%) |
| | 256 | 49204 | 0 | 0 | 49204 | 49204(100%) | 553 | 10698 | 0 | 0 | 10698 | 10698(100%) |
| | 258 | 55621 | 0 | 0 | 55621 | 55621(100%) | 554 | 14002 | 0 | 0 | 14002 | 14002(100%) |
| | 284 | 46562 | 0 | 0 | 46562 | 46562(100%) | 575 | 14373 | 0 | 0 | 14373 | 14373(100%) |
| | 288 | 22916 | 0 | 0 | 22916 | 22916(100%) | 582 | 17559 | 0 | 0 | 17559 | 17559(100%) |
| | 289 | 17928 | 0 | 0 | 17928 | 17928(100%) | 584 | 23013 | 0 | 0 | 23013 | 20148(100 %) |
| | 290 | 26859 | 0 | 0 | 26859 | 26859(100%) | 586 | 25502 | 0 | 0 | 25502 | 25502(100%) |
| | 295 | 32534 | 0 | 0 | 32534 | 32534(100%) | 587 | 18379 | 0 | 0 | 18379 | 18379(100%) |
| | 301 | 20127 | 0 | 0 | 20127 | 20127(100%) | 589 | 22951 | 0 | 0 | 22951 | 22951(100%) |
| | 306 | 15473 | 2 | 0 | 15471 | 15471(100%) | 590 | 19561 | 0 | 0 | 19561 | 19561(100%) |
| | 31.1 | 16179 | 0 | 0 | 16179 | 16179(100%) | | | | | | |

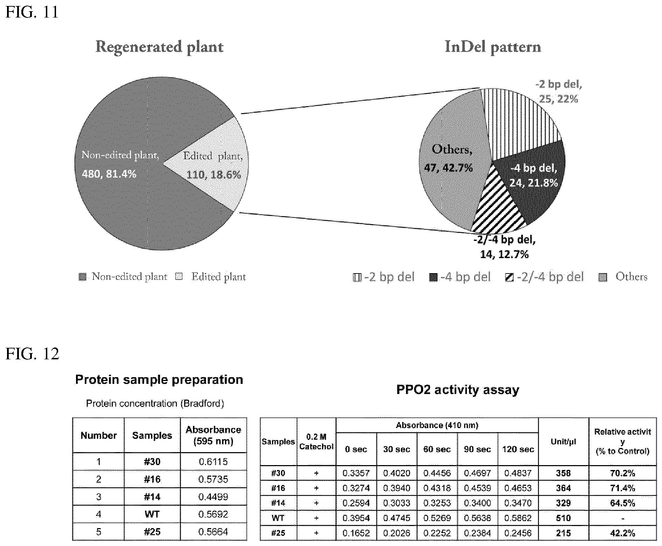

As a result of deep-sequencing of the above 110 plants, in particular, it was found that, in the plant with edited *StPPO2* gene produced by the method of the present invention, the mutation in which -2 bp or -4 bp deletion pattern is shown from all of the four alleles is responsible for 57% (63/110) of the InDel pattern of the entire 110 edited plants (Table 5 and Fig. 11).

**Table 5**

| Pattern of InDel mutation in 110 plants with edited *StPPO2* gene | | | | |
|---|---|---|---|---|
| InDel Pattern # | Deletion size (bp) | Number of plants | Deletion sites (Base position from PAM sequence) | Remarks |
| 1 | -2 | 25 | 5^{th}, 6^{th} | All 4 alleles were found to have -2 bp deletion pattern |
| 2 | -4 | 24 | 4^{th}, 5^{th}, 6^{th}, 7^{th} | All 4 alleles were found to have -4 bp deletion pattern |
| 3 | -2/-4 | 14 | pattern 1 & 2 | Among 4 alleles, each 2 alleles were found to have number 1 and number 2 deletion pattern, respectively |
| 4 | -1 | 47 | 4^{th} | Among 4 alleles, with number 1 and number 2 deletion pattern, an allele having -1 bp deletion pattern was found |
| | -5 | | 4^{th}, 5^{th}, 6^{th}, 7^{th}, 8^{th} | Among 4 alleles, with number 1 and number 2 deletion pattern, an allele having -5 bp deletion pattern was found |
| | -10 | | 5^{th} ∼ 14^{th} | Only from 1 allele of #205 line, -10 bp deletion pattern was found |
| Total | | 110 | | |

To finally obtain a tuber from the potato lines having edited *StPPO2* gene, young potato plants which have been found to have edited gene were cultured after transferring them to a greenhouse for cultivating seed potato. After 3 months or so, a tuber was obtained from some of the potato lines. PPO2 protein activity and suppression of browning were then observed using the tuber.

### Example 5. Measurement of PPO2 protein activity in plant with edited StPPO2

PPO2 activity measurement from tissues of a potato plant which has been found to have edited *StPPO2* gene (#14, 16, 25 and 30) and also a non-edited control (WT) potato was carried out according to the following method. First, 0.5 g of potato tuber was chopped to small pieces and added to a 1.5 ml tube. Cold sodium phosphate solution (pH 6.5, 1 ml) was added thereto and the tissues were homogenized using a pipette tip. After that, the mixture was centrifuged for 20 minutes at 13,000 rpm, 4°C condition. While carrying out the centrifuge, sodium phosphate solution (pH 5.5) and 0.2 M pyrocatechol solution were admixed with each other in a 96-well plate and subjected to pre-incubation for 10 minutes. After the above centrifuge, the supernatant was added to the 96-well plate which has been added with a mixture solution of sodium phosphate and pyrocatechol followed by pre-incubation for 10 minutes, and the absorbance at 410 nm was immediately measured. The absorbance measurement was carried out up to 2 minutes with an interval of 30 seconds starting from sec 0.

According to Bradford assay, protein concentration was measured by using the remaining supernatant. Based on the measured protein concentration and the absorbance value measured at 410 nm, units of PPO2 were calculated.

For a blind test to avoid any experimental bias, plant samples with edited gene were numbered, and then the test was carried out as described in the above. According to the result of measuring the PPO2 protein activity, it was found that, in the plant line with edited gene, the PPO2 activity was suppressed at least by 28.6% and at most by 57.8% compared to the non-edited control (WT) as the result is illustrated in FIG. 12.

### Example 6. Examination of browning of plant with edited StPPO2

Observation of potato browning was made with the following 2 methods.

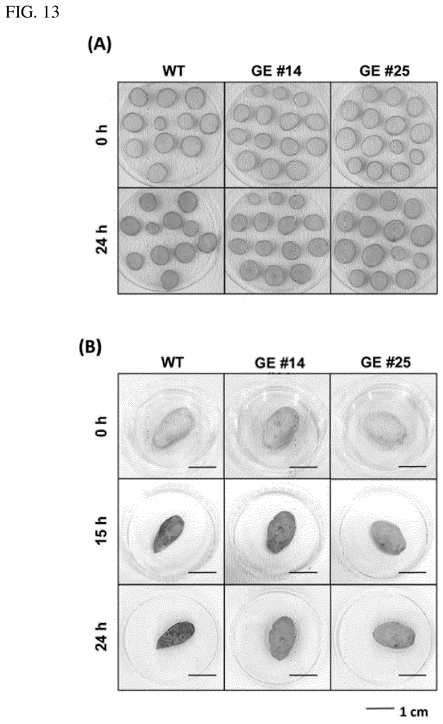

First, a potato tuber was sliced using the same method as the method employed in the previous report (Gonzalez MN et al., Front Plant Sci. (2020) 10: 1649), and used for the test. The result is shown in FIG. 13(A). As a result of observing browning after slicing the potato tuber, it was found that the browning is suppressed compared to the control (WT). However, due to the moisture evaporation, there was no significant progress in the suppression of browning so that a potato tuber with the same size as above was simply peeled and used for the observation of browning. As a result, if was found that the tuber of a potato plant having edited *StPPO2* (GE#14, # 25) has significantly suppressed browning compared to the control (WT) (FIG. 13(B)). Consequently, it was possible to recognize that the browning of a plant line with edited *StPPO2* gene is related to the activity of PPO2 protein.

## Claims

1. A composition for genome editing to suppress browning in a potato plant comprising, as an active ingredient, an ribonucleoprotein complex of a guide RNA specific to a target nucleotide sequence in *StPPO2* (*Solanum tuberosum* Polyphenol Oxidase 2) gene derived from potato and an endonuclease protein; or a recombination vector comprising a DNA encoding a guide RNA specific to a target nucleotide sequence in *StPPO2* gene derived from potato and a nucleic acid sequence encoding an endonuclease protein.

2. The composition according to Claim 1, wherein the target nucleotide sequence in *StPPO2* gene consists of the nucleotide sequence of SEQ ID NO: 5.

3. A guide nucleic acid as an RNA sequence specific to a target region in nucleotide sequence constituting *StPPO2* gene in which the target region is the nucleotide sequence of SEQ ID NO: 5.

4. A method of producing a genome-edited potato plant with suppressed browning including:
(a) introducing a guide RNA specific to a target nucleotide sequence in *StPPO2* gene derived from potato and an endonuclease protein to a potato plant cell to have genome editing; and
(b) regenerating a potato plant from the potato plant cell that is genome-edited.

5. The method according to Claim 4, wherein the introduction of a guide RNA and an endonuclease protein to a potato plant cell in the step (a) uses an ribonucleoprotein complex of a guide RNA specific to a target nucleotide sequence in *StPPO2* gene derived from potato and an endonuclease protein; or a recombination vector comprising a DNA encoding a guide RNA specific to a target nucleotide sequence in *StPPO2* gene derived from potato and a nucleic acid sequence encoding an endonuclease protein.

6. The method according to Claim 4, wherein the target nucleotide sequence in *StPPO2* gene consists of the nucleotide sequence of SEQ ID NO: 5.

7. The method according to Claim 4, wherein the potato plant cell that is genome-edited of the step (b) has been induced to have deletion mutation of 1 to 10 bp in size in the target nucleotide sequence in *StPPO2* gene.

8. A genome-edited potato plant with suppressed browning which is produced by the method of any one of Claims 4 to 7.

9. The genome-edited potato plant according to Claim 8, wherein the genome-edited potato plant with suppressed browning has been induced to have deletion mutation of 1 to 10 bp in size in a target nucleotide sequence in *StPPO2* gene.

10. Genome-edited seed potato of the potato plant according to Claim 8.
